# EUROPEAN PATENT APPLICATION

(11) **EP 2 596 829 A1**
(43) Date of publication of application: **29.05.2013**
(21) Application number: 11809685.8
(22) Date of filing: 21.07.2011
(51) Int. Cl.: A61M 25/04

(54) **INTRAVASCULAR DRUG ELUTION-TYPE CATHETER**

(30) Priority: 23.07.2010 JP 2010165553
(71) Applicant: Nipro Corporation, Osaka-shi, Osaka 531-8510 (JP)
(72) Inventor: TANIKAWA, Masahiro, Osaka-shi Osaka 531-8510 (JP)
(74) Representative: Bertsch, Florian Oliver
(86) International application number: PCT/JP2011/066511
(87) International publication number: WO 2012/011515

(57) **Abstract**

An intravascular drug elution-type catheter 10, has: an elastically deformable portion 11 provided with a plurality of wire members 33 that are disposed apart from each other in the circumferential direction, maintaining an inflated shape in a basket shape in which the wire members 33 are apart from each other and the outer diameter of the central portion 34 in the longitudinal direction 101 is enlarged, and elastically deforming into a deflated shape in which the wire members 33 come close to each other, so that the outer diameter of the central portion 34 is reduced; a shaft 12 provided with the elastically deformable portion 11 at the top end side thereof; a catheter body 13 having a lumen 20 through which the elastically deformable portion 11 in the deflated shape and the shaft 12 can pass; a drug holding film 15 which is stretched only at the central portion 34 of the elastically deformable portion 11 in the circumferential direction relative to the wire members 33 in the inflated shape and which has flexibility with which the drug holding film 15 follows the change in the shape of the elastically deformable portion 11; and a drug held in the drug holding film 15.

## Description

### Technical Field

The present invention relates to a catheter capable of eluting a drug in a blood vessel.

### Background Art

Heretofore, medical treatment has been performed which includes inserting a catheter into a blood vessel, and then locally giving a drug. For example, in percutaneous transluminal coronary angioplasty (PTCA), a stenosis portion of the coronary arteries is expanded with a balloon catheter, and then, in order to prevent re-narrowing of the coronary arteries, a drug is infiltrated into a blood vessel wall. As an instrument for infiltrating a drug into a blood vessel wall, a balloon catheter is known in which a balloon is impregnated with a drug (Patent document 1 and 2).

### Citation List

### [Patent Documents]

[Patent Document 1] Japanese Unexamined Patent Application Publication (Translation of PCT Application) No. 2010-509991
[Patent document 2] Japanese Unexamined Patent Application Publication (Translation of PCT Application) No. 2005-510315

### Summary of Invention

### Problems to be Solved by the Invention

In the operation using the balloon catheter described above, by inserting the balloon catheter into a blood vessel wall into which a drug is to be infiltrated, inflating the balloon, and then pressing the inflated balloon against the blood vessel wall for several minutes, the drug impregnated in the balloon is eluted to be infiltrated into the blood vessel wall. However, blocking the blood flow toward the peripheral side relative to the balloon by the inflated balloon may induce tissue damages, such as a myocardial necrosis, and therefore it is not preferable. In contrast, when a period of time for maintaining the state where the balloon is inflated is shortened, a sufficient amount of the drug cannot be infiltrated into the blood vessel wall.

The present invention has been made in view of such a situation, and aims to provide a unit capable of directly infiltrating a drug into a blood vessel wall without blocking the blood flow toward the peripheral side.

### Means for Solving the Problems

(1) An intravascular drug elution-type catheter according to the invention, has: an elastically deformable portion provided with a plurality of wire members that are disposed apart from each other in the circumferential direction, maintaining an inflated shape in a basket shape in which the wire members are apart from each other and the outer diameter of the central portion in the longitudinal direction is enlarged, and elastically deforming into a deflated shape in which the wire members come close to each other, so that the outer diameter of the central portion is reduced; a shaft provided with the elastically deformable portion at the top end side thereof; a catheter body having a first lumen through which the elastically deformable portion in the deflated shape and the shaft can pass; a drug holding film which is stretched only at the central portion of the elastically deformable portion in the circumferential direction relative to the wire members in the inflated shape, which has flexibility with which the drug holding film follows the change in the shape of the elastically deformable portion, and which can hold and elute a drug; and the drug held in the drug holding film.

The intravascular drug elution-type catheter is inserted into the blood vessel after a stenosis portion was medically treated, for example. When the intravascular drug elution-type catheter is inserted into a blood vessel, the elastically deformable portion is formed into a deflated shape and is accommodated in the first lumen of the catheter body. The drug holding film is also deflated following the deflation of the elastically deformable portion and is accommodated in the first lumen. The catheter body whose elastically deformable portion is accommodated in the first lumen is inserted into a blood vessel along a guide wire with the elastically deformable portion side as the top end.

When the top end side of the catheter body reaches near the stenosis portion after medically treated, the shaft is inserted into the catheter body, and then the elastically deformable portion is pushed out from the first lumen of the catheter body. The pushed-out elastically deformable portion elastically returns to the inflated shape from the deflated shape, and maintains the inflated shape. With the shape change of the elastically deformable portion, i.e., with the shape change of the elastically deformable portion into a basket shape, the drug holding film is stretched in the circumferential direction relative to the wire members. This drug holding film presses the blood vessel wall at the stenosis portion after medically treated. By holding the pressing of the drug holding film and the blood vessel wall for a predetermined time, the drug held in the drug holding film is eluted to be infiltrated into the blood vessel wall.

The drug holding film is stretched only at the central portion of the elastically deformable portion in the circumferential direction relative to the wire members. The central portion is a portion serving as the center in the longitudinal direction of the intravascular drug elution-type catheter in the elastically deformable portion. In other words, at the top end portion and the proximal end portion of the elastically deformable portion, the drug holding film is not stretched in the circumferential direction to the wire members. Therefore, at the top end portion and at the proximal end portion, the blood can circulate between the wire members. In the basket-shaped elastically deformable portion, the blood can circulate through the internal space. Therefore, in the state where the elastically deformable portion maintains the inflated shape, the blood can circulate from the top end portion to the proximal end portion and from the proximal end portion to the top end portion.

(2) The drug holding film is bonded to the wire members at least from the central portion to the top end portion of the elastically deformable portion and regions between the wire members may be cut at the top end portion.

When the elastically deformable portion accommodated in the first lumen of the catheter body is pushed out to the outside, it is assumed that the drug holding film slides to the inner wall of the catheter body in the first lumen. Due to the fact that the drug holding film is bonded to the wire members from the central portion to the top end portion of the elastically deformable portion, position shift of the drug holding film relative to the wire members of the elastically deformable portion due to such sliding is hard to occur. Since the regions between the wire members are cut at the top end portion of the elastically deformable portion, the circulation of the blood is not hindered by the drug holding film at the top end portion.

(3) As the elastically deformable portion, one is mentioned in which the wire members spirally extend in the longitudinal direction, both ends of the wire members are united, and the diameter of the central portion is the maximum diameter.

(4) The shaft is a tubular body through which the guide wire can pass. As the elastically deformable portion, one is mentioned through which the guide wire can pass inside in the diameter direction relative to the wire members.

(5) The catheter body has a second lumen through which the guide wire can pass. As the elastically deformable portion, one is mentioned through which the shaft can pass inside in the diameter direction relative to the wire members.

### Effects of the Invention

According to the intravascular drug elution-type catheter of the invention, when the intravascular drug elution-type catheter is inserted into a blood vessel, the elastically deformable portion is formed into a deflated shape and accommodated in the first lumen of the catheter body. When the top end side of the catheter body reaches near a damaged blood vessel wall, the shaft is inserted into the catheter body, the elastically deformable portion is pushed out from the first lumen of the catheter body to elastically return to an inflated shape from the deflated shape, and then the drug holding film presses the blood vessel wall. Thus, the drug eluted from the drug holding film can be infiltrated into the blood vessel wall.

Since the drug holding film is stretched only at the central portion of the elastically deformable portion in the circumferential direction relative to the wire members, the blood can circulate from the top end portion to the proximal end portion and from the proximal end portion to the top end portion in the state where the elastically deformable portion maintains the inflated shape. Thus, the state where the drug holding film is pressed against the blood vessel wall can be maintained without blocking the blood flow toward the peripheral side.

### Brief Description of Drawings

[Fig. 1] Fig. 1 is s view illustrating the appearance configuration of an intravascular drug elution-type catheter 10 in a state where an elastically deformable portion 11 is in an inflated shape.
[Fig. 2] Fig. 2 is an enlarged view of the intravascular drug elution-type catheter 10 in a region II of Fig. 1.
[Fig. 3] Fig. 3 is a cross sectional view of an elastically deformable portion 11 along the III-III line of Fig. 2.
[Fig. 4] Fig. 4 is a cross sectional view of the intravascular drug elution-type catheter 10 in a state where the elastically deformable portion 11 is in a deflated shape.
[Fig. 5] Fig. 5 is a schematic view illustrating a state of inserting the intravascular drug elution-type catheter 10 into a blood vessel 90 by forming the elastically deformable portion 11 into a deflated shape.
[Fig. 6] Fig. 6 is a schematic view illustrating a state where a drug is infiltrated into a stenosis portion 92 by forming the elastically deformable portion 11 into an inflated shape.
[Fig. 7] Fig. 7 is an enlarged view illustrating a modification of the intravascular drug elution-type catheter 10.

### Modes for Carrying out the Invention

Hereinafter, a preferable embodiment of the invention is described. This embodiment is merely one embodiment of the invention, and it is a matter of course that embodiment can be modified in the range where the scope of the invention is not altered.

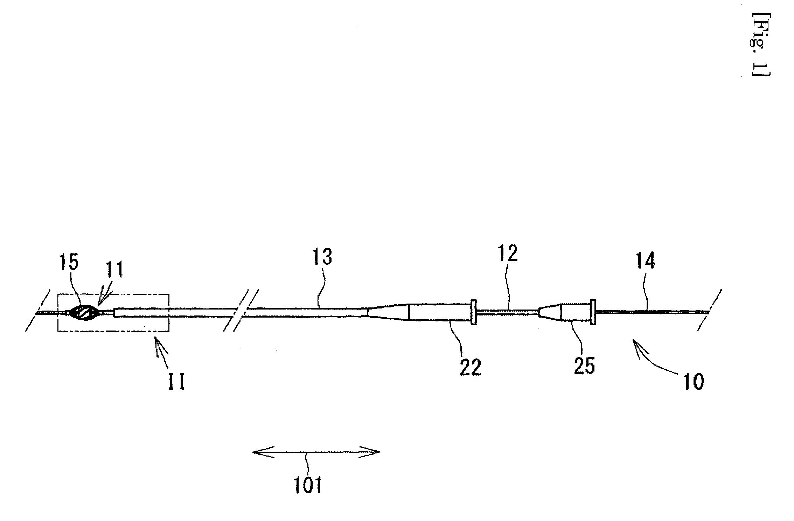

As illustrated in Fig. 1, the intravascular drug elution-type catheter 10 according to this embodiment has a shaft 12 provided with an elastically deformable portion 11 at the top end side and a catheter body 13 into which the shaft 12 is inserted.

The catheter body 13 has a tube 21 having a lumen 20 (first lumen) as the main structure. The tube 21 is a molded body of a soft plastic which can elastically deform, such as polyamide and polyether amide. The tube 21 has substantially the same outer diameter over a longitudinal direction 101. The lumen 20 also has substantially the same inner diameter over the longitudinal direction 101. The inner diameter of the lumen 20 is adjusted to a diameter which allows the elastically deformable portion 11 and the shaft 12 to pass. The length in the longitudinal direction 101 of the tube 21 is adjusted as appropriate considering the length from a catheter insertion portion, such as the human limbs, to an affected portion.

At the proximal end of the tube 21, a handle portion 22 is provided. The handle portion 22 is a cylindrical member having an internal space continuous to the lumen 20. The handle portion 22 is a molded body of resin, such as polypropylene and ABS. The handle portion 22 can serve as a grip in operation of insertion and removal of the tube 21. Although not illustrated in Fig. 1, the handle portion 22 may be provided with a blade as appropriate in order to facilitate rotation operation around the axial direction 101. In this embodiment, the proximal end side refers to the rear side to the direction where the intravascular drug elution-type catheter 10 is inserted into the body. The top end side refers to the front side to the direction where the intravascular drug elution-type catheter 10 is inserted into the body.

The shaft 12 is a thin and long tube made from stainless steel for medical treatment. The shaft 12 may be coated with fluororesin or the like. The internal space of the shaft 12 allows a guide wire 14 to pass. The shaft 12 can elastically deform along a curve shape of the arterial blood vessel and the like and has rigidity with which the shaft 12 does not buckle in the longitudinal direction 101.

To the proximal end of the shaft 12, a handle portion 25 is connected. The handle portion 25 is a cylindrical member having an internal space continuous the internal space of the shaft 12. The handle portion 25 is a molded body of resin, such as polypropylene and ABS. The handle portion 25 can serve as a grip when inserting/removing the shaft 12 into/from the catheter body 13 or inserting/removing the guide wire 14 into/from the shaft 12.

The elastically deformable portion 11 is provided at the top end of the shaft 12. The elastically deformable portion 11 has a fixed ring 31, a slide ring 32, and a plurality of wire members 33. The fixed ring 31 is a cylindrical member into which the guide wire 14 can be inserted. The fixed ring 31 is fixed to the top end of the shaft 12, and the internal space is continuous to the internal space of the shaft 12.

The slide ring 32 is disposed apart from the fixed ring 31 at the top end side in the longitudinal direction 101. The slide ring 32 is a cylindrical member into which the guide wire 14 can be inserted. The internal space of the slide ring 32 is disposed in such a manner as to be on the same axis as that of the internal space of the fixed ring 31. Therefore, the guide wire 14 which is inserted into the internal space of the fixed ring 31 to be extended to the top end side in the longitudinal direction 101 along the longitudinal direction 101 can be inserted into the internal space of the slide ring 32.

The wire members 33 are stretched between the fixed ring 31 and the slide ring 32. The wire members 33 connect the fixed ring 31 and the slide ring 32. The wire members 33 are disposed apart from each other in a circumferential direction 102 around the guide wire 14 extended along the longitudinal direction 101. The spaced interval between the wire members 33 is adjusted to the size which allows the blood to smoothly circulate through the space between the adjacent wire members 33, and specifically, is about several millimeters.

Each wire member 33 is spirally extended in the longitudinal direction 101 between the fixed ring 31 and the slide ring 32 and united by the fixed ring 31 and the slide ring 32. Thus, the elastically deformable portion 11 defined by each wire member 33 presents the outer diameter in a basket shape in which the outer diameter of the central portion 34 is the maximum diameter. The guide wire 14 is inserted into the internal space in the basket shape, i.e., inside in the diameter direction 103 of each wire member 33.

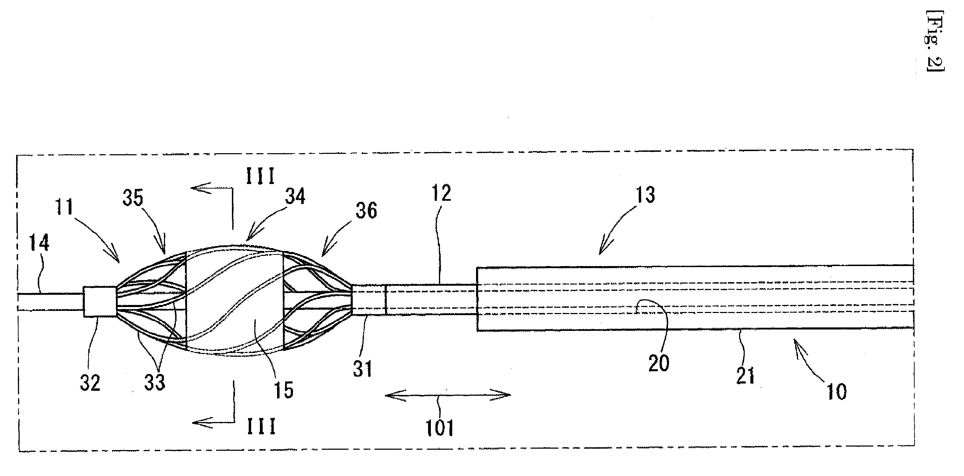

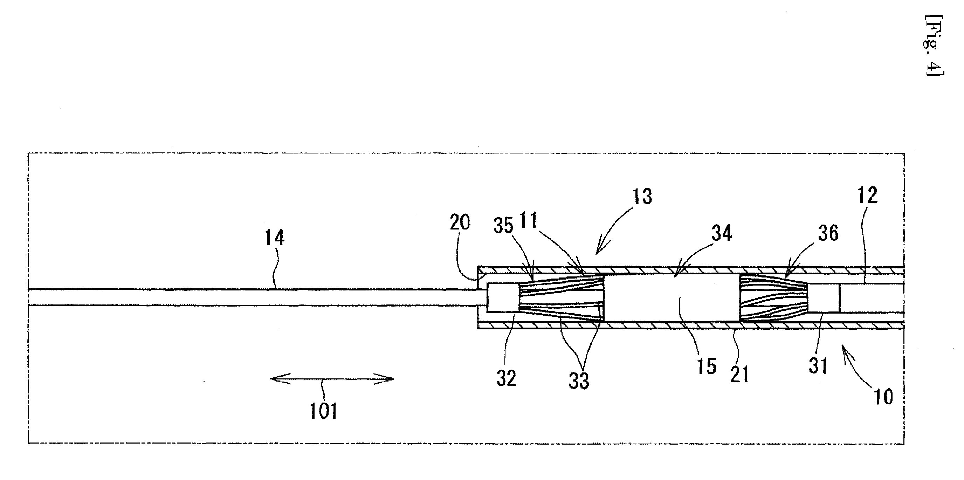

As a material of each wire member 33, a shape memory alloy is preferable and, specifically, a Ni-Ti alloy, a Cu-Zn-Al alloy, a Cu-Al-Ni alloy, and the like are mentioned. By the shape memorized by each wire member 33, the elastically deformable portion 11 is maintained in the inflated shape in the basket shape where the wire members 33 are spaced from each other and the outer diameter of the central portion 34 is enlarged as illustrated in Fig. 2. Then, when external force inside in the diameter direction, i.e., the central side into which the guide wire 14 is inserted, acts on each wire member 33, each wire member 33 comes close to each other, so that the elastically deformable portion 11 elastically deforms into a deflated shape where the outer diameter of the central portion 34 decreases as illustrated in Fig. 4.

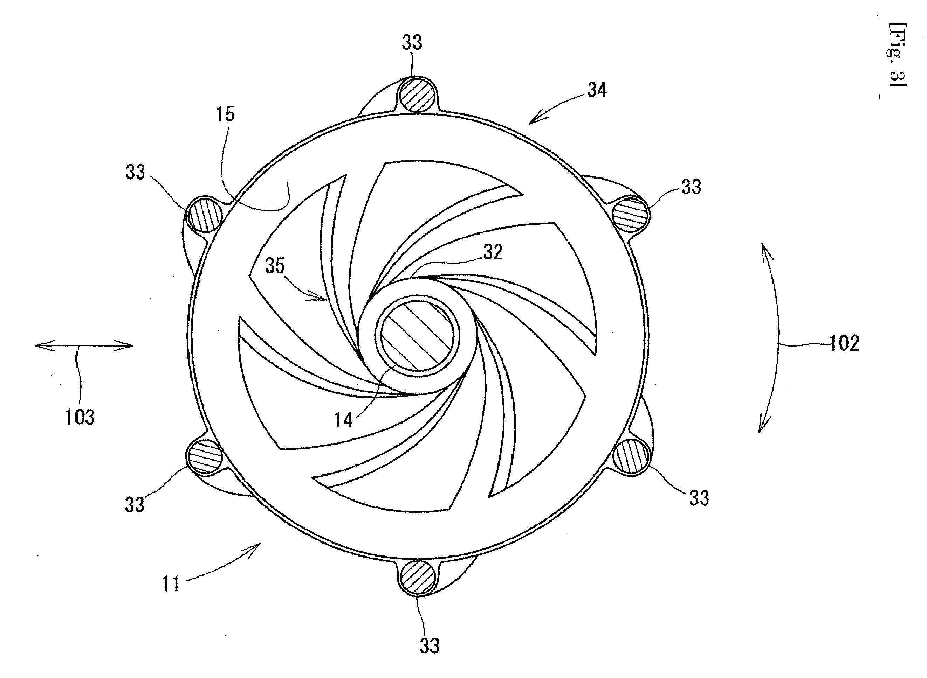

As illustrated in Figs. 2 and 3, the drug holding film 15 is stretched only at the central portion 34 of the elastically deformable portion 11 in the circumferential direction 102 relative to the wire members 33. In other words, the drug holding film 15 is not stretched between the wire members 33 at the top end portion 35 and the proximal end portion 36 of the elastically deformable portion 11.

As a material of the drug holding film 15, materials having biocompatibility and capable of holding a desired drug and eluting the held drug in a blood vessel are preferable, and, specifically, polyurethane, polyethylene, polyester, polypropylene, polyamide, polytetrafluoroethylene, polyvinylidene fluoride, and the like are mentioned. The drug holding film 15 is stretched with moderate tension between the wire members 33 at the central portion 34 when the elastically deformable portion 11 is in the inflated shape. The drug holding film 15 has flexibility, and therefore when the elastically deformable portion 11 is in the deflated shape, the drug holding film 15 bends between the wire members 33 to follow the shape change of the elastically deformable portion 11.

The drug holding film 15 is stretched between the wire members 33 by the elastically deformable portion 11 being immersed in an solution serving as the material of the drug holding film 15, and then dried. In this case, by only the top end portion 35 and the central portion 34 of the elastically deformable portion 11 being immersed in a solution, the drug holding film 15 is stretched between the wire members 33 in a state where the drug holding film 15 is bonded to the wire members 33 at portions other than a proximal end portion 36, i.e., at the top end portion and the central portion 34. Thereafter, at the top end portion 35 of the elastically deformable portion 11, the drug holding film 15 stretched between the wire members 33 is cut by laser. As a result, as illustrated in Fig. 3, at the top end portion 35 and the proximal end portion 36, the drug holding film 15 is not present between the wire members 33, and then the drug holding film 15 is stretched between the wire members 33 only at the central portion 34.

The drug holding film 15 is coated with a drug. The drug is not particularly limited. Mentioned as a drug for preventing re-narrowing after expanding a stenosis portion of the coronary arteries, paclitaxel and analogues thereof, rapamycin and analogues thereof, β-lapachone and analogues thereof, biological vitamin D and analogues thereof, and the like.

A layer coated with the drug holding film 15 may contain an additive and the like for the purpose of, for example, enhancing discharge of the drug in a blood vessel, besides the drugs mentioned above. On the layer of the drug, a protective layer for preventing loss of the drug while passing through a blood vessel may be further laminated. The drug may be held in the drug holding film 15 not only by coating the drug holding film 15 with the drug but also infiltrating the drug into the drug holding film 15.

### [Method for using intravascular drug elution-type catheter 10]

Hereinafter, a method for using the intravascular drug elution-type catheter 10 is described.

The intravascular drug elution-type catheter 10 is inserted into the blood vessel 90 having a blood vessel wall 91 to be medically treated. The blood vessel wall 91 is the stenosis portion 92 expanded in the percutaneous transluminal coronary angioplasty (PTCA), for example, and is the stenosis portion 92 into which a drug is infiltrated in order to prevent re-narrowing.

Before the intravascular drug elution-type catheter 10 is inserted into the blood vessel 90, the guide wire 14 is inserted into the blood vessel 90 to reach the blood vessel wall 91. Such insertion of the guide wire 14 is achieved by known techniques disclosed in Japanese Unexamined Patent Application Publication Nos. 2006-326226 and 2006-230442, for example, and therefore detailed explanation is omitted here.

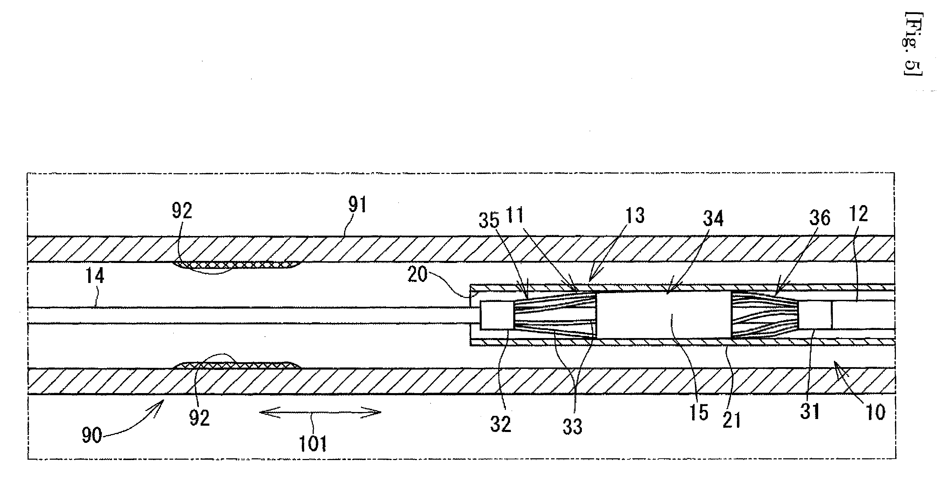

When the intravascular drug elution-type catheter 10 is inserted into the blood vessel 90, the elastically deformable portion 11 is formed into a deflated shape, and accommodated in the lumen 20 of the catheter body 13 as illustrated in Fig. 5. The drug holding film 15 follows the deflation of the elastically deformable portion 11 to be deflated, and is also accommodated in the lumen 20. The proximal end of the guide wire 14 is inserted into the slide ring 32 of the elastically deformable portion 11 from the top end side of the intravascular drug elution-type catheter 10 in this state, i.e., the top end side of the catheter body 13. The guide wire 14 is further inserted into the fixed ring 31 from the internal space of the elastically deformable portion 11, and inserted into the internal space of the shaft 12. Then, the intravascular drug elution-type catheter 10 is inserted into the blood vessel 90 along the guide wire 14.

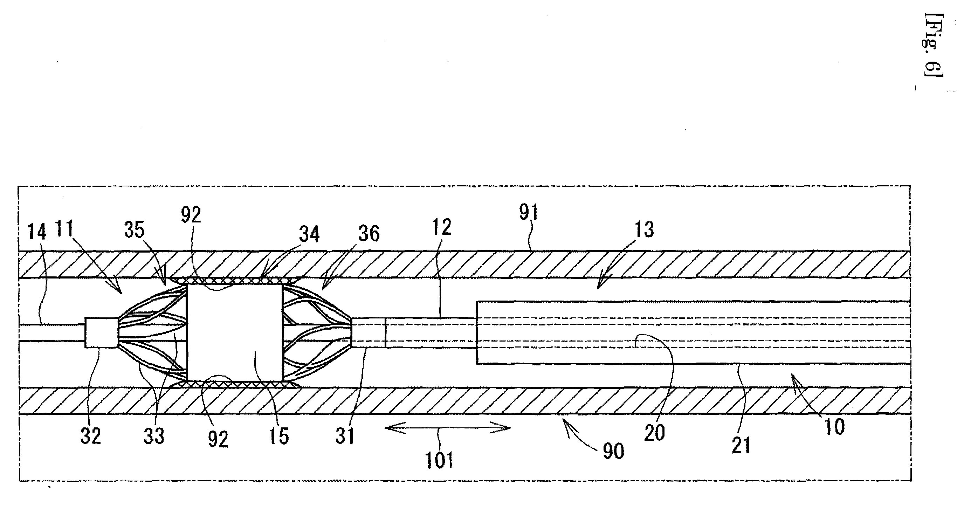

When the top end side of the catheter body 13 reaches near the stenosis portion 92 of the blood vessel wall 91 as illustrated in Fig. 5, the handle portions 22 and 25 are operated, so that the shaft 12 is inserted into the catheter body 13. More specifically, the shaft 12 is relatively pushed out to the top end side relative to the catheter body 13. Thus, the elastically deformable portion 11 is pushed out from the lumen 20 of the catheter body 13. As illustrated in Fig. 6, the pushed-out elastically deformable portion 11 elastically returns to an inflated shape from the deflated shape, and maintains the inflated shape. With the shape change of the elastically deformable portion 11, i.e., with the shape change of the elastically deformable portion 11 into a basket shape, the drug holding film 15 is stretched in the circumferential direction relative to the wire members 33. The drug holding film 15 presses the stenosis portion 92 of the blood vessel wall 91. By holding pressing of the stenosis portion 92 by the drug holding film 15 for a predetermined time, the drug is infiltrated into the stenosis portion 92 from the drug holding film 15.

The drug holding film 15 is stretched only at the central portion 34 of the elastically deformable portion 11 in the circumferential direction relative to the wire members 33 and the drug holding film 15 is not present between the wire members 33 at the top end portion 35 and the proximal end portion 36. Therefore, while the elastically deformable portion 11 in the inflated shape is left in the blood vessel 90, the blood flowing through the blood vessel 90 can circulate through the elastically deformable portion 11 from the top end portion 35 to the proximal end portion 36 or from the proximal end portion 36 to the top end portion 35. It is a matter of course that the blood can circulate also through the internal space of the basket-shaped elastically deformable portion 11. Therefore, while the drug holding film 15 is pressed against the stenosis portion 92 by the elastically deformable portion 11 in the inflated shape, the blood flow is not blocked due to blocking of the blood vessel 90.

When a predetermined time for infiltrating the drug into the stenosis portion 92 passes, the handle portions 22 and 25 are operated, so that the shaft 12 is drawn out relative to the catheter body 13. More specifically, the shaft 12 is relatively drawn back to the proximal end side relative to the catheter body 13. Thus, the elastically deformable portion 11 in an inflated shape is drawn into the lumen 20 of the catheter body 13. In the elastically deformable portion 11, the wire members 33 are extended in such a manner as to continuously spread to the outside in the diameter direction 103 from the fixed ring 31 to the proximal end portion 36 and the central portion 34. Therefore, the wire members 33 are elastically deformed to the inside in the diameter direction 103 with the top end of the catheter body 13 as a guide. Thus, the elastically deformable portion 11 is accommodated in the lumen 20 of the catheter body 13 while the shape of the elastically deformable portion 11 gradually changes from the inflated shape to the deflated shape. The drug holding film 15 follows the shape change of the elastically deformable portion 11 to bend between the wire members 33 from the state where the drug holding film 15 is stretched between the wire members 33 at the central portion 34. Then, when the elastically deformable portion 11 is completely accommodated in the lumen 20 of the catheter body 13, the intravascular drug elution-type catheter 10 is drawn out from the blood vessel 90.

### [Effects of this embodiment]

According to the intravascular drug elution-type catheter 10 of this embodiment, when the intravascular drug elution-type catheter 10 is inserted into the blood vessel 90, the elastically deformable portion 11 is formed into a deflated shape and accommodated in the lumen 20 of the catheter body 13. When the top end side of the catheter body 13 reaches near the stenosis portion 92 of the blood vessel wall 91, the shaft 12 is inserted into the catheter body 13, the elastically deformable portion 11 is pushed out from the lumen 20 of the catheter body 13 to elastically return to an inflated shape from the deflated shape, and then the drug holding film 15 presses the stenosis portion 92. Thus, the drug held in the drug holding film 15 can be infiltrated into the stenosis portion 92.

Since the drug holding film 15 is stretched only at the central portion 34 of the elastically deformable portion 11 in the circumferential direction 102 relative to the wire members 33, the blood can circulate from the top end portion 35 to the proximal end portion 36 and from the proximal end portion 36 to the top end portion 35 in a state where the elastically deformable portion 11 maintains the inflated shape. Thus, the shape in which the elastic deformation part 11 is inflated can be maintained in the blood vessel 90 for a desired time without blocking the blood flow toward the peripheral side.

The drug holding film 15 is stretched between the wire members 33 from the central portion 34 to the top end portion 35 of the elastically deformable portion 11, and then the drug holding film 15 between the wire members 33 is cut at the top end portion 35. Therefore, even when the drug holding film 15 slides to the inner wall of the catheter body 13 in the lumen 20 when the elastically deformable portion 11 accommodated in the lumen 20 of the catheter body 13 is pushed out to the outside, position shift of the drug holding film 15 to the wire members 33 of the elastically deformable portion 11 is difficult to occur. Moreover, since the regions between the wire members 33 are cut at the top end portion 35 of the elastically deformable portion 11, the circulation of the blood is not hindered by the drug holding film 15 at the top end portion 35.

### [Modification]

Hereinafter, a modification of the above-described embodiment is described. In the above-described embodiment, the catheter body 13 is an over-the-wire-type. In contrast thereto, in this modification, the catheter body 13 is a rapid-exchange type and is different from the above-described embodiment in that the guide wire 14 is not inserted into the shaft 41 and the guide wire 14 is inserted into a catheter body 42 of a so-called double lumen type. The elastically deformable portion 11 has the same structure as that of the above-described embodiment, and the same reference numerals as those of the above-described embodiment are given.

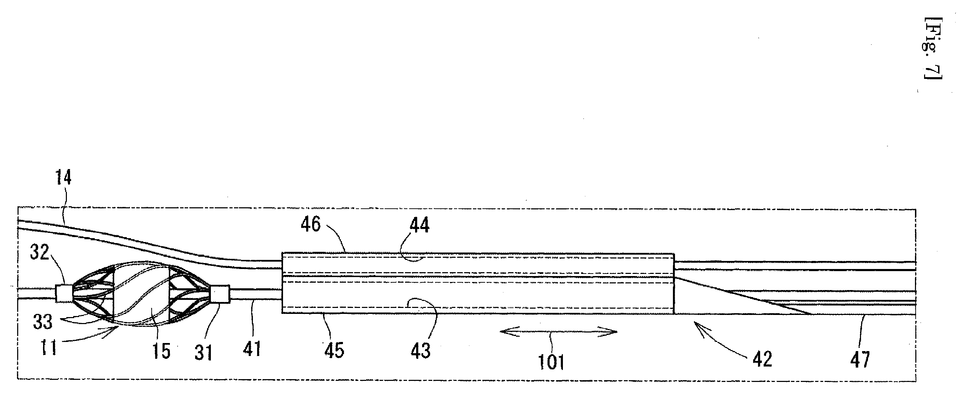

As illustrated in Fig. 7, the catheter body 42 has two lumens 43 and 44. The lumen 43 (first lumen) is formed by the internal space of a tube 45. The lumen 44 (second lumen) is formed by the internal space of a tube 46. The two tubes 45 and 46 are connected in a state where the lumens 43 and 44 are disposed parallel to each other. Into the lumen 43, the shaft 41 is inserted. Into the lumen 44, the guide wire 14 is inserted.

The length in the longitudinal direction 101 of the tubes 45 and 46 is sufficiently short to the distance from the insertion position to the affected portion in the blood vessel 90. Therefore, in the tube 46, only a portion of guide wire 14 is inserted into the lumen 44.

To the proximal end of the tube 45, a catheter shaft 47 is connected and is extended in the longitudinal direction 101. The catheter shaft 47 is a stainless steel wire member for medical treatment and has elasticity with which the catheter shaft 47 elastically bends along a curve shape of the blood vessel 90 and rigidity with which the catheter shaft 47 does not buckle in the longitudinal direction 101. Although not illustrated in Fig. 7, the same handle portion as the handle portion 22 is provided at the proximal end side of the catheter shaft 47.

The shaft 41 is a stainless steel wire member for medical treatment. The shaft 41 can elastically deform along a curve shape of the blood vessel 90 and has rigidity with which the shaft 41 does not buckle in the longitudinal direction 101. The elastically deformable portion 11 is provided at the top end side of the shaft 41. The top end side of the shaft 41 is inserted into the fixed ring 31 and the slide ring 32 of the elastically deformable portion 11. The fixed ring 31 is fixed to the shaft 41. The slide ring 32 is slidable to the shaft 41.

In the lumen 43 of the tube 45, the elastically deformable portion 11 in a deflated shape can be accommodated. Then, when the shaft 41 is relatively pushed out to the top end side relative to the catheter body 42, the elastically deformable portion 11 is pushed out from the lumen 43 of the catheter body 42. Then, as illustrated in Fig. 7, the pushed-out elastically deformable portion 11 elastically returns to an inflated shape from the deflated shape, and maintains the inflated shape. When the shaft 41 is relatively drawn back to the proximal end side relative to the catheter body 42, the elastically deformable portion 11 in the inflated shape is drawn into the lumen 43 of the catheter body 42 while changing the shape into a deflated shape. By such a modification, the same effects as those in the above-described embodiment are demonstrated.

### Description of Reference Numerals

| | |
|---|---|
| 10 | Intravascular drug elution-type catheter |
| 11 | Elastically deformable portion |
| 12, 41 | Shaft |
| 13, 42 | Catheter body |
| 14 | Guide wire |
| 15 | Drug holding film |
| 20, 43 | Lumen (first lumen) |
| 33 | Wire member |
| 34 | Central portion |
| 35 | Top end portion |
| 44 | Lumen (second lumen) |

## Claims

1. An intravascular drug elution-type catheter, comprising:
an elastically deformable portion provided with a plurality of wire members that are disposed apart from each other in the circumferential direction, maintaining an inflated shape in a basket shape in which the wire members are apart from each other and the outer diameter of the central portion in the longitudinal direction is enlarged, and elastically deforming into a deflated shape in which the wire members come close to each other, so that the outer diameter of the central portion is reduced;
a shaft provided with the elastically deformable portion at the top end side thereof;
a catheter body having a first lumen through which the elastically deformable portion in the deflated shape and the shaft can pass;
a drug holding film which is stretched only at the central portion of the elastically deformable portion in the circumferential direction relative to the wire members in the inflated shape, which has flexibility with which the drug holding film follows the change in the shape of the elastically deformable portion, and which can hold and elute a drug; and
the drug held in the drug holding film.

2. The intravascular drug elution-type catheter according to Claim 1, wherein the drug holding film is bonded to the wire members at least from the central portion of the elastically deformable portion to the top end portion thereof and regions between the wire members are cut at the top end portion.

3. The intravascular drug elution-type catheter according to Claim 1 or 2, wherein the elastically deformable portion is one in which the wire members are spirally extended in the longitudinal direction, both ends of the wire members are united, and the diameter of the central portion is the maximum diameter.

4. The intravascular drug elution-type catheter according to any one of Claims 1 to 3, wherein
the shaft is a tubular body through which the guide wire can pass, and
the elastically deformable portion is one through which the guide wire can pass inside in the diameter direction relative to the wire members.

5. The intravascular drug elution-type catheter according to any one of Claims 1 to 3, wherein
the catheter body has a second lumen through which the guide wire can pass, and
the elastically deformable portion is one through which the shaft can pass inside in the diameter direction relative to the wire members.
